# FASCICULE DE BREVET EUROPEEN

(11) **EP 4 232 127 B1**
(45) Date de publication et mention de la délivrance du brevet: **25.09.2024**
(21) Numéro de dépôt: 21806310.5
(22) Date de dépôt: 20.10.2021
(51) Int. Cl.: A61M 11/00, A61M 15/00, A61M 15/08, B05B 11/02, B05B 11/00

(54) **DISPOSITIF DE DISTRIBUTION DE PRODUIT FLUIDE**
VORRICHTUNG ZUR ABGABE EINES FLÜSSIGEN PRODUKTS
DEVICE FOR DISPENSING A FLUID PRODUCT

(30) Priorité: 20.10.2020 FR 2010765
(43) Date de publication de la demande: 30.08.2023
(73) Titulaire: APTAR France SAS, 27110 Le Neubourg (FR)
(72) Inventeur: GRAINE, Lila, 78650 Beynes (FR)
(74) Mandataire: CAPRI
(86) Numéro de dépôt international: PCT/FR2021/051835
(87) Numéro de publication internationale: WO 2022/084631

(56) Documents cités:
- WO-A1-2014/147329
- WO-A1-2019/241832
- WO-A1-2020/099766
- US-A- 5 431 155

## Description

La présente invention concerne un dispositif de distribution de produit fluide, notamment de type bidose.

Par dispositif de distribution de type bidose, on entend un dispositif contenant deux doses de produit fluide à distribuer lors de deux actionnements successifs du dispositif de distribution.

Les dispositifs de type bidose sont bien connus dans l'état de la technique. Ces dispositifs comportent généralement un réservoir contenant les deux doses de produit fluide à distribuer, et un organe de distribution, qui est généralement un piston, qui est monté coulissant dans ledit réservoir, et qui est déplacé pour distribuer le produit fluide contenu dans ledit réservoir. Le déplacement du piston se fait en deux courses d'actionnement successives, de sorte qu'une première dose est distribuée lors d'un premier actionnement et une seconde dose est distribuée lors d'un deuxième actionnement.

Avec ce type de dispositif de type bidose, il est parfois difficile pour l'utilisateur de savoir s'il a distribué une ou deux dose(s) avec son dispositif. Or, selon le type de produit fluide qui est distribué par le dispositif, notamment lorsqu'il s'agit d'un médicament, il peut être important d'éviter tout risque de sous-dosage et/ou de surdosage. Ainsi, par exemple, si le dispositif de type bidose est destiné à distribuer une dose respective dans chaque narine, il n'est généralement pas souhaitable que les deux doses soient distribuées dans la même narine. Or, un utilisateur qui aurait utilisé le dispositif pour distribuer une première dose dans sa première narine, et qui l'aurait ensuite reposé ou qui aurait été distrait, risquerait, s'il n'est pas certain d'avoir déjà utilisé le dispositif une première fois, de distribuer la seconde dose dans la même narine que la première dose. Ceci n'est généralement pas souhaitable. Ainsi, si l'on expulse deux fois le produit dans la même narine, l'excédent d'actif ne sera pas correctement absorbé par les tissus, voire repartira immédiatement de la narine en coulant, avec une perte évidente d'efficacité. De plus, aucune dose ne sera alors disponible pour la seconde narine. Par ailleurs, avec certains produits fluides, comme les vaccins, il peut être souhaitable de pouvoir vérifier l'état du produit fluide avant distribution, notamment pour vérifier que la formulation conserve son intégrité, par exemple que le produit fluide est clair et ne contient pas de précipité. Ceci requiert une fenêtre de visualisation suffisamment grande et non obstruée par des parties du dispositif, comme par exemple le ressort.

Il a été proposé des dispositifs comportant un indicateur de dose, pour indiquer à l'utilisateur la distribution des doses successives. Le document WO2014147329 décrit un tel indicateur. Un inconvénient des indicateurs existants est leur taille relativement petite, pas toujours facile à voir, notamment pour des personnes âgées ou dont la vision est dégradée.

Les documents WO2019241832, WO2020099766, US5431155, FR2625981, US6708846, EP0580897 et US2005015051 décrivent d'autres dispositifs de l'état de la technique.

La présente invention a pour but de fournir un dispositif de distribution de produit fluide qui ne reproduit pas les inconvénients susmentionnés.

La présente invention a ainsi pour but de fournir un dispositif de distribution de produit fluide qui fournit un indicateur de grande taille et non obstrué par des parties du dispositif, comme par exemple le ressort.

La présente invention a également pour but de fournir un tel dispositif de distribution de produit fluide qui soit simple et peu coûteux à fabriquer et à assembler.

La présente invention a donc pour objet un dispositif de distribution de produit fluide comportant un réservoir contenant au moins deux doses de produit fluide, un organe de distribution formé par un piston monté coulissant dans ledit réservoir pour distribuer du produit fluide, une tête de distribution pourvue d'un orifice de distribution et d'une jupe latérale, un élément repose-doigts étant prévu sur ladite tête de distribution, ledit dispositif comportant un organe d'actionnement axialement déplaçable à l'intérieur de ladite jupe latérale pour réaliser les actionnements successifs de dispositif en déplaçant ledit réservoir par rapport à ladite tête de distribution pour ainsi déplacer ledit piston dans ledit réservoir et ainsi distribuer du produit fluide à travers ledit orifice de distribution, un ressort de rappel étant prévu pour ramener ledit organe d'actionnement dans sa position de départ après chaque actionnement, ledit dispositif comportant un indicateur comportant une fenêtre de visualisation et des moyens d'indication, ladite fenêtre de visualisation étant disposée dans ladite jupe latérale, sous ledit élément repose-doigts, ledit ressort de rappel coopérant avec un épaulement radial d'un manchon central fixé dans ladite tête de distribution, ledit épaulement radial étant disposé axialement en-dessous de ladite fenêtre de visualisation, de sorte que ledit ressort de rappel n'est jamais visible dans ladite fenêtre de visualisation.

Avantageusement, ledit réservoir contient deux doses de produit fluide, distribuées lors de deux actionnements successifs du dispositif.

Avantageusement, lesdits moyens d'indication comportent au moins une zone d'indication en couleur, ladite zone d'indication en couleur apparaissant partiellement dans ladite fenêtre de visualisation après distribution de la première dose de produit fluide et totalement après distribution de la seconde dose de produit fluide.

Avantageusement, ladite zone d'indication en couleur dudit indicateur est formée sur un corps fixé audit réservoir.

En variante, ladite zone d'indication en couleur dudit indicateur est formée sur ledit réservoir.

Avantageusement, ledit manchon central comporte une paroi au moins partiellement transparente et/ou une ou plusieurs découpes, pour permettre de voir lesdits moyens d'indication à travers ladite fenêtre de visualisation.

Avantageusement, ledit indicateur est adapté à indiquer une distribution de dose incomplète à travers ladite fenêtre de visualisation.

Ces avantages et caractéristiques et d'autres de la présente invention apparaitront plus clairement au cours de la description détaillée suivante, faite en référence aux dessins joints, donnés à titre d'exemples non limitatifs, et sur lesquels

Les figures 1, 2 et 3 sont des vues schématiques de côté d'un dispositif de distribution de produit fluide selon un mode de réalisation avantageux, respectivement avant distribution de la première dose, après distribution de la première dose, et après distribution de la seconde dose,

La figure 4 est une vue schématique en section transversale du dispositif de distribution des figures 1 à 3, en position de repos avant la distribution de la première dose,

La figure 5 est une vue similaire à celle de la figure 4, après distribution de la première dose,

La figure 6 est une vue similaire à celle de la figure 5, avant distribution de la seconde dose,

La figure 7 est une vue similaire à celle de la figure 6, après distribution de la seconde dose, et

La figure 8 est une vue similaire à celle de la figure 7, en fin de cycle d'actionnement.

Dans la description ci-après, les termes "axial" et "radial" se réfèrent à l'axe central longitudinal du dispositif.

La présente invention sera décrite ci-après en référence à un exemple de réalisation qui est un bidose, c'est-à-dire un dispositif contenant deux doses de produit fluide à distribuer lors de deux actionnements successifs du dispositif. Il est toutefois entendu que la présente invention pourrait s'appliquer à des dispositifs comportant un nombre supérieur de doses, par exemple trois ou quatre doses. De même, le dispositif de type bidose représenté sur les dessins n'est qu'un exemple de réalisation possible auquel la présente invention s'applique, et il est entendu que la présente invention s'applique de manière plus générale à tout type de dispositif contenant au moins deux doses.

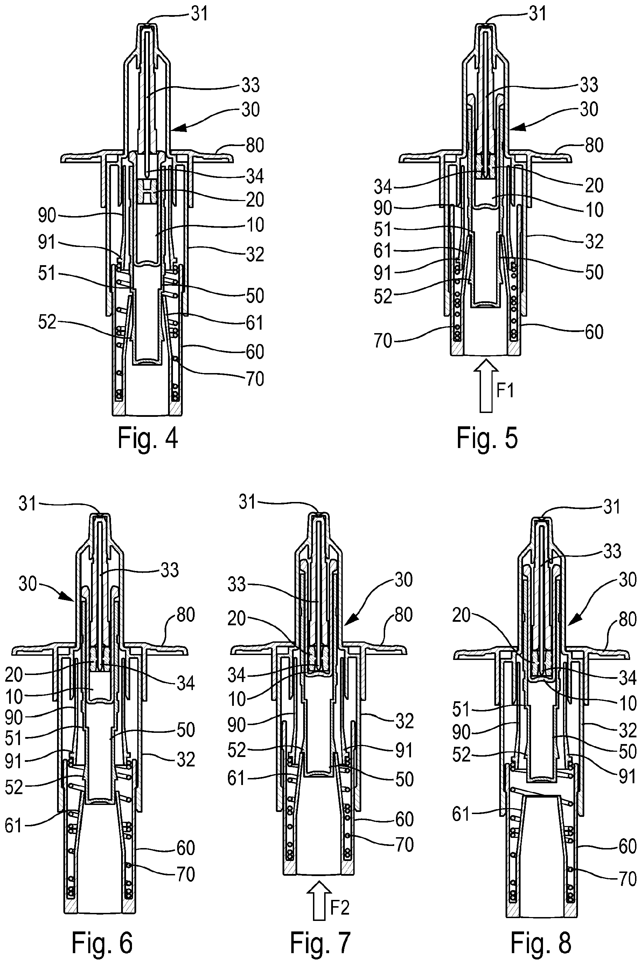

En se référant aux figures, le dispositif de distribution est un bidose qui comporte un réservoir 10 contenant deux doses de produit fluide. Un organe de distribution formé par un piston 20 est monté coulissant dans ledit réservoir 10. Dans la position avant actionnement du dispositif, représentée sur la figure 4, ledit piston 20 fait office de bouchon en isolant le contenu du réservoir 10.

Une tête de distribution 30 est assemblée sur ledit réservoir 10 en étant déplaçable axialement par rapport à celui-ci. En particulier, un déplacement axial de la tête de distribution 30 par rapport au réservoir 10 provoque le déplacement du piston 20 dans le réservoir 10 et ainsi la distribution du produit fluide contenu dans ledit réservoir. La tête de distribution 30 comporte un canal de distribution 33 qui mène depuis une pointe de percement 34 jusqu'à un orifice de distribution 31 de la tête de distribution 30. En amont de l'orifice de distribution 31, il peut être prévu un profil de pulvérisation, qui peut être d'un quelconque type connu et qui n'est pas représenté plus en détail sur les dessins, pour distribuer le produit fluide sous forme de spray.

Plus précisément, dans l'exemple représenté, le réservoir 10 est fixé dans un corps 50, qui est donc solidaire dudit réservoir 10, et qui se déplace ensemble avec lui.

La tête de distribution 30 comporte une jupe latérale inférieure 32 adaptée à coopérer avec un organe d'actionnement 60. Un élément repose-doigts 80 est assemblé autour de ladite tête de distribution 30, ou en variante être formé de manière monobloc avec elle.

Ledit organe d'actionnement 60 est axialement déplaçable à l'intérieur de ladite jupe latérale 32 de la tête de distribution 30 pour réaliser les actionnements successifs de dispositif. Comme visible sur les figures 4 à 8, l'organe d'actionnement 60 comporte au moins une patte inclinée 61 qui est adaptée à coopérer avec des projections 51, 52 du corps 50 pour réaliser les actionnements successifs.

Un ressort de rappel 70 est monté entre l'organe d'actionnement 60 et un manchon central 90 fixé dans la tête de distribution 30, pour ramener ledit organe d'actionnement 60 dans sa position de départ après chaque actionnement.

Le fonctionnement du dispositif représenté sur les figures 4 à 8 est le suivant. Dans la position de repos de la figure 4, le piston bouchon 20 isole le contenu du réservoir 10 de l'atmosphère. Lorsque l'utilisateur appuie simultanément sur le repose-doigt 80 et sur l'organe d'actionnement 60, il va déplacer ledit organe d'actionnement 60 à l'intérieur de la jupe latérale 32 de la tête de distribution 30, selon la flèche F1 sur la figure 5. Ceci va pousser le corps 50 axialement vers le haut à partir de la position représentée sur la figure 4, par l'intermédiaire des pattes 61 qui vont pousser sur l'épaulement 51 dudit corps. Ceci va comprimer le ressort 70 et déplacer le réservoir 10 par rapport à la tête de distribution 30. Lorsque le réservoir 10 va commencer à se déplacer par rapport à la tête de distribution 30, l'extrémité de percement 34 du canal de distribution 33 va venir percer le piston de bouchon 20 pour faire communiquer l'intérieur du réservoir 10 avec ledit canal d'expulsion 33. Une poursuite de l'actionnement va provoquer un déplacement du piston 20 à l'intérieur du réservoir et donc une distribution de la première dose. Le produit fluide est donc poussé par ledit piston 20 à travers l'extrémité de percement 34 dans le canal de distribution 33, puis hors du dispositif à travers l'orifice de distribution 31.

Après distribution de la première dose, le dispositif est dans la position représentée sur la figure 5, et lorsque l'utilisateur relâche l'organe d'actionnement 60, le ressort 70 va ramener celui-ci vers sa position de départ. Pendant ce retour de l'organe d'actionnement 60, le réservoir et le corps 50 ne reviennent pas en arrière, car ces deux composants restent maintenus dans ladite tête de distribution 30. Eventuellement, la tête de distribution 30 peut comporter des moyens anti-retour pour empêcher un retour dudit corps 50 et/ou dudit réservoir 10 en arrière. Lorsque l'organe d'actionnement 60 revient sous l'effet du ressort de rappel 70 vers sa position de repos, les pattes 61 vont se positionner sous la deuxième projection 52 du corps 50 ce qui va permettre à l'utilisateur d'actionner une deuxième fois le dispositif pour distribuer la deuxième dose de produit fluide, selon la flèche F2 sur la figure 7.

Les figures 6 et 7 représentent respectivement les positions avant et après distribution de la deuxième dose.

Le dispositif de distribution comporte un indicateur 40 pour indiquer à l'utilisateur la distribution de la première dose et la distribution de la deuxième dose. De cette manière, l'utilisateur sait exactement où il en est et si la première dose a ou non été distribuée. Cet indicateur 40 comporte une fenêtre de visualisation 35 formée dans ladite jupe latérale 32 de ladite tête de distribution 30, axialement sous l'élément repose-doigts 80. Avantageusement, cette fenêtre de visualisation 35 est formée dans ladite jupe latérale 32 de la tête 30 en étant bien visible de l'utilisateur lorsqu'il tient le dispositif dans sa main. Cette fenêtre de visualisation 35 peut notamment être réalisées sous la forme d'une ouverture traversante à travers la jupe latérale 32 de la tête de distribution 30.

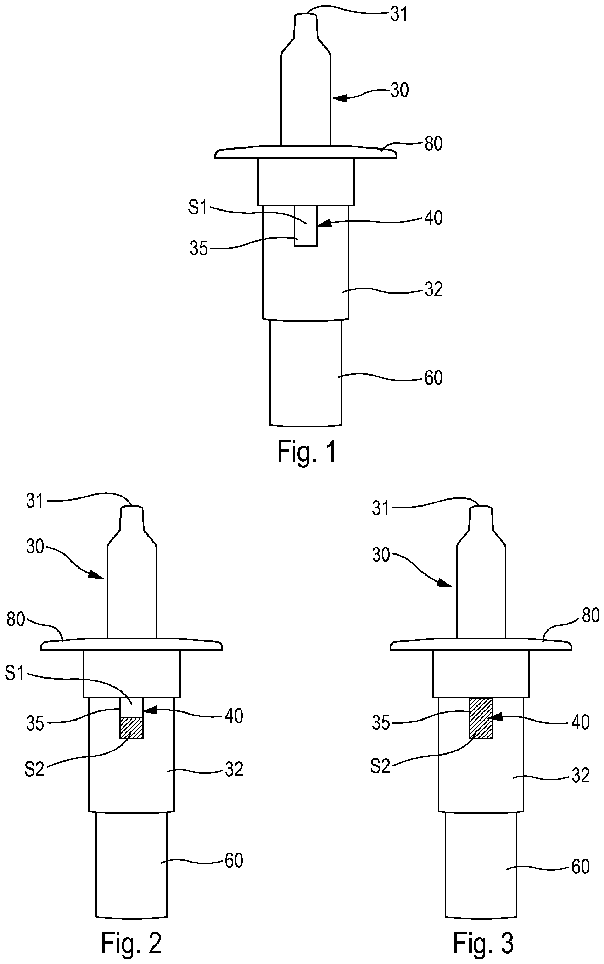

Ledit indicateur 40 comporte en outre des moyens d'indications S2, tels qu'une zone de couleur, qui sont formés de préférence sur ledit corps 50 qui est fixé au réservoir 10. Le manchon central 90, disposé autour dudit corps 50, comporte une paroi au moins partiellement transparente, et/ou une ou plusieurs découpes, pour permettre de voir lesdits moyens d'indication S2 affichés dans la fenêtre de visualisation 35. Toutefois, en variante on pourrait imaginer des moyens d'indication S2 formés directement sur le réservoir 10, auquel cas le corps 50 devrait aussi comporter des parties transparentes ou découpées. Ces moyens d'indication S2 deviennent progressivement visibles dans la fenêtre de visualisation 35, comme illustré sur les figures 1 à 3. Avant la première dose, la fenêtre de visualisation 35 montre une première surface S1 dans une première couleur, comme visible sur la figure 1. Après distribution de la première dose, une partie de la fenêtre de visualisation 35 montre une seconde surface S2 réalisée dans une seconde couleur différente de la première couleur, de sorte que la fenêtre de visualisation 35 montre dans cette position deux surfaces S1, S2 de couleurs différentes, comme visible sur la figure 2. Après distribution de la seconde dose, la totalité de la fenêtre de visualisation 35 montre la seconde surface S2 dans la seconde couleur, comme visible sur la figure 3.

Avantageusement, l'indicateur 40 permet aussi d'indiquer une distribution incomplète, notamment en dimensionnant de manière appropriée la fenêtre de visualisation 35 et les moyens d'indication. Ainsi, seule la distribution d'une première dose complète permet à l'indicateur d'afficher dans la fenêtre de visualisation des première et seconde surfaces S1, S2 de dimensions équivalentes. Une distribution incomplète sera donc détectable par l'utilisateur grâce à une seconde surface S2 de dimension inférieure à la première surface S1 dans la fenêtre de visualisation 35.

Selon l'invention, ledit manchon central 90 comporte un épaulement radial 91 coopérant avec le bord supérieur dudit ressort 70, ledit épaulement radial 91 étant disposé axialement en-dessous de ladite fenêtre de visualisation 35, de sorte que jamais le ressort 70 n'est visible dans ladite fenêtre de visualisation. Cette mise en oeuvre permet à la fois de prévoir une fenêtre de visualisation de grande dimension, facilitant la visualisation de l'indication par l'utilisateur, et d'éviter que la présence du ressort dans la fenêtre ne perturbe cette indication. Ceci permet notamment de s'assurer de l'intégrité du produit fluide, par exemple de l'absence de précipité dans le produit fluide, avant distribution d'une dose.

Bien entendu, la présente invention a été décrite en référence à un mode de réalisation, qui n'est pas limitatif, et toute modification utile peut être apportée à la présente invention sans sortir du cadre de celle-ci tel que défini par les revendications annexées.

## Revendications

1. Dispositif de distribution de produit fluide comportant un réservoir (10) contenant au moins deux doses de produit fluide, un organe de distribution formé par un piston (20) monté coulissant dans ledit réservoir (10) pour distribuer du produit fluide, une tête de distribution (30) pourvue d'un orifice de distribution (31) et d'une jupe latérale (32), un élément repose-doigts (80) étant prévu sur ladite tête de distribution, ledit dispositif comportant un organe d'actionnement (60) axialement déplaçable à l'intérieur de ladite jupe latérale (32) pour réaliser les actionnements successifs de dispositif en déplaçant ledit réservoir (10) par rapport à ladite tête de distribution (30) pour ainsi déplacer ledit piston (20) dans ledit réservoir (10) et ainsi distribuer du produit fluide à travers ledit orifice de distribution (31), un ressort de rappel (70) étant prévu pour ramener ledit organe d'actionnement (60) dans sa position de départ après chaque actionnement, ledit dispositif comportant un indicateur (40) comportant une fenêtre de visualisation (35) et des moyens d'indication (S2), **caractérisé en ce que** ladite fenêtre de visualisation (35) est disposée dans ladite jupe latérale (32), sous ledit élément repose-doigts (80), ledit ressort de rappel (70) coopérant avec un épaulement radial (91) d'un manchon central (90) fixé dans ladite tête de distribution (30), ledit épaulement radial (91) étant disposé axialement en-dessous de ladite fenêtre de visualisation (35), de sorte que ledit ressort de rappel (70) n'est jamais visible dans ladite fenêtre de visualisation (35).

2. Dispositif selon la revendication 1, dans lequel ledit réservoir (10) contient deux doses de produit fluide, distribuées lors de deux actionnements successifs du dispositif.

3. Dispositif selon la revendication 2, dans lequel lesdits moyens d'indication comportent au moins une zone d'indication en couleur (S2), ladite zone d'indication en couleur (S2) apparaissant partiellement dans ladite fenêtre de visualisation (35) après distribution de la première dose de produit fluide et totalement après distribution de la seconde dose de produit fluide.

4. Dispositif selon la revendication 3, dans lequel ladite zone d'indication en couleur (S2) dudit indicateur (40) est formée sur un corps (50) fixé audit réservoir (10).

5. Dispositif selon la revendication 3, dans lequel ladite zone d'indication en couleur (S2) dudit indicateur (40) est formée sur ledit réservoir (10).

6. Dispositif selon l'une quelconque des revendications précédentes, dans lequel ledit manchon central (90) comporte une paroi au moins partiellement transparente et/ou une ou plusieurs découpes, pour permettre de voir lesdits moyens d'indication (S2) à travers ladite fenêtre de visualisation (35).

7. Dispositif selon l'une quelconque des revendications précédentes, dans lequel ledit indicateur (40) est adapté à indiquer une distribution de dose incomplète à travers ladite fenêtre de visualisation (35).

## Patentansprüche

1. Vorrichtung zur Abgabe eines flüssigen Produkts, die einen Behälter (10) aufweist, der mindestens zwei Dosen eines flüssigen Produkts enthält, ein Abgabeorgan, das von einem Kolben (20) gebildet wird, der gleitend in dem Behälter (10) montiert ist, um das flüssige Produkt abzugeben, einen Abgabekopf (30), der mit einer Abgabeöffnung (31) und einer Seitenschürze (32) versehen ist, wobei ein Fingerauflageelement (80) auf dem Abgabekopf vorgesehen ist, und die Vorrichtung ein Betätigungsorgan (60) aufweist, das im Inneren der Seitenschürze (32) axial verschiebbar ist, um die aufeinanderfolgenden Betätigungen der Vorrichtung durchzuführen, indem der Behälter (10) in Bezug auf den Abgabekopf (30) verschoben wird, um so den Kolben (20) in dem Behälter (10) zu verschieben und so das flüssige Produkt durch die Abgabeöffnung (31) abzugeben, wobei eine Rückstellfeder (70) vorgesehen ist, um das Betätigungsorgan (60) nach jeder Betätigung in seine Ausgangsposition zurückzubringen, wobei die Vorrichtung eine Anzeige (40) mit einem Sichtfenster (35) und Anzeigemitteln (S2) aufweist, **dadurch gekennzeichnet, dass** das Sichtfenster (35) in der Seitenschürze (32) unter dem Fingerauflageelement (80) angeordnet ist, wobei die Rückstellfeder (70) mit einer radialen Schulter (91) einer zentralen Hülse (90) zusammenwirkt, die in dem Abgabekopf (30) befestigt ist, wobei die radiale Schulter (91) axial unterhalb des Sichtfensters (35) angeordnet ist, so dass die Rückstellfeder (70) in dem Sichtfenster (35) niemals sichtbar ist.

2. Vorrichtung nach Anspruch 1, wobei der Behälter (10) zwei Dosen eines flüssigen Produkts enthält, die bei zwei aufeinanderfolgenden Betätigungen der Vorrichtung abgegeben werden.

3. Vorrichtung nach Anspruch 2, wobei die Anzeigemittel mindestens einen farbigen Anzeigebereich (S2) aufweisen, wobei der farbige Anzeigebereich (S2) in dem Sichtfenster (35) nach der Abgabe der ersten Dosis des flüssigen Produkts teilweise und nach der Abgabe der zweiten Dosis des flüssigen Produkts vollständig erscheint.

4. Vorrichtung nach Anspruch 3, wobei der farbige Anzeigebereich (S2) der Anzeige (40) auf einem Körper (50) ausgebildet ist, der an dem Behälter (10) befestigt ist.

5. Vorrichtung nach Anspruch 3, wobei der farbige Anzeigebereich (S2) der Anzeige (40) auf dem Behälter (10) ausgebildet ist.

6. Vorrichtung nach einem der vorhergehenden Ansprüche, wobei die zentrale Hülse (90) eine zumindest teilweise transparente Wand und/oder einen oder mehrere Ausschnitte aufweist, um die Anzeigemittel (S2) durch das Sichtfenster (35) sehen zu können.

7. Vorrichtung nach einem der vorhergehenden Ansprüche, wobei die Anzeige (40) dazu geeignet ist, eine unvollständige Dosisabgabe durch das Sichtfenster (35) anzuzeigen.

## Claims

1. A fluid dispenser device comprising a reservoir (10) containing at least two doses of fluid, a dispensing member constituted by a piston (20) that is mounted to slide in said reservoir (10) so as to dispense fluid, a dispensing head (30) that is provided with a dispensing orifice (31) and a side skirt (32), a finger-rest element (80) being provided on said dispensing head, said device comprising an actuating member (60) which is axially movable inside said side skirt (32) in order to carry out successive actuations of the device by displacing said reservoir (10) relative to said dispensing head (30) so as to thus displace said piston (20) in said reservoir (10) and thus dispense fluid through said dispensing orifice (31), a return spring (70) being provided to return said actuating member (60) to its starting position after each actuation, said device comprising an indicator (40) comprising a viewing window (35) and indicator means (S2), **characterised in that** said viewing window (35) is disposed in said side skirt (32) under said finger-rest element (80), said return spring (70) co-operating with a radial shoulder (91) of a central sleeve (90) fixed in said dispensing head (30), said radial shoulder (90) being disposed axially below said viewing window (35), such that said return spring (70) is never visible in said viewing window (35).

2. The device according to claim 1, wherein said container (10) contains two doses of fluid, dispensed during two successive actuations of the device.

3. The device according to claim 2 said indicator means include at least one colored indication zone (S2), said colored indication zone (S2) partially appearing in said viewing window (35) after the first dose of fluid has been dispensed, and fully after the second dose of fluid has been dispensed.

4. The device according to claim 3, wherein said colored indication zone (S2) of said indicator (40) is formed on a body (50) fastened to said reservoir (10).

5. The device according to claim 3, wherein said colored indication zone (S2) of said indicator (40) is formed on said reservoir (10).

6. The device according to any one of the preceding claims, wherein said central sleeve (90) includes an at least partially transparent wall and/or one or more cutouts, to enable said indicator means (S2) to be seen through said viewing window (35).

7. The device according to any one of the preceding claims, wherein said indicator (40) is adapted to indicate through said viewing window (35) that an incomplete dose has been dispensed.
